# EUROPEAN PATENT APPLICATION

(11) **EP 1 522 305 A1**
(43) Date of publication of application: **13.04.2005**
(21) Application number: 05075041.3
(22) Date of filing: 28.11.2000
(51) Int. Cl.: A61K 31/50, A61P 25/00

(54) **Combinations of sumatriptan with a cyclooxygenase II inhibitor**

(30) Priority: 08.12.1999 GB 9929039
(62) Divisional of application: 00979767.1
(71) Applicant: GLAXO GROUP LIMITED, Greenford, Middlesex UB6 ONN (GB)
(72) Inventor: Gutterman, Donna, Research Triangle Park, NC 27709 (US); Salonen, Reijo Juhani, Research Triangle Park, NC 27709 (US)
(74) Representative: Reed, Michael Antony

(57) **Abstract**

A method of treating conditions associated with cephalic pain and alleviating the symptoms associated therewith which comprises administering to a mammal, including man, sumatriptan or a pharmaceutically acceptable salt or solvate thereof and 2-(4-ethoxy-phenyl)-3-(4-methanesulfonyl-phenyl)-pyrazolo[1,5-b]pyridazine or a phamaceutically acceptable salt or solvate thereof.

## Description

The present invention relates to the treatment of conditions associated with cephalic pain such as migraine, cluster headache, chronic paroxysmal hemicrania, headache associated with vascular disorders, headache associated with substances or their withdrawal and tension headache. In particular it relates to the use of a 5HT₁ agonist in conjunction with a COX-2 inhibitor.

5-HT₁-like receptors are located, for example, in the dog saphenous vein and the 5-HT₁-like receptor agonists with which the present invention is concerned contract the dog saphenous vein. Such compounds may therefore be identified by their contractile effect on the dog isolated saphenous vein strip as described, for example, by Apperley et al., Br. J. Pharmacol, 68, 215-224 (1980). Compounds which are selective 5-HT₁-like receptor agonists have also been found to selectively constrict the carotid arterial bed of the anaesthetised dog.

A variety of compounds which selectively constrict the dog isolated saphenous vein strip and which constrict the carotid arterial bed of the anaesthetised dog have been described in the art. These include indole derivatives such as those disclosed inter alia in published British Patent Specifications Nos. 2082175, 2081717, 2083463, 2124210, 2150932, 2162522, 2168347, 2168973, 2185020, 2186874, 2191488, 2208646, 2289464, 2289465, 2286185, published European Patent Specifications Nos. 147107, 237678, 242939, 244085, 225726, 254433, 303506, 313397, 354777, 382570, 464558, 506363, 506369, 450238, 451022, 451008, 478954, 438230, 494774, 497512, 501568, 580539, 581538, 586866, 590970, 590971, 603432, 610134, 620222, 620223, 644187, 641787, 645385, 648767, 666258, 668273, 683155, 700905, 703229, 707007, 708102, 712837, 714894, 714896, 729958, 733628, 736525, 747353, 749962, 755932, 768301, 810220, and published International patent application Nos. WO92/11013, W092/11014, WO92/06973, WO93/00086, WO92/13856, WO93/00094, WO91/18897, WO93/00333, WO94/02460, WO94/02477, WO94/03446, WO94/06789, WO94/10171, WO94/11363, WO94/11356, WO94/13620, WO94/14770, WO94/14771, WO94/14772, WO94/14773, WO94/14779, WO94/15930, WO94/18193, WO94/20466, WO94/21610, WO94/21611, WO94/21619, WO94/24127, WO94/29293, WO95/01334, WO95/01965, WP95/05366, WO95/05381, WO95/05383, WO95/06636, WO95/11903, WO95/20588, WO95/21166, WO95/21167, WO95/28933, WO95/32196, WO96/04269, WO96/04274, WO96/06638, WO96/06846, WO96/09288, WO96/11195, WO96/11685, WO96/11923, WO96/11930, WO96/12721, WO96/12713, WO96/16056, WO96/16949, WO96/16961, WO96/17831, WO96/17842, WO96/23784, WO96/23785, WO96/23789, WO96/24596, WO96/29075, WO96/39133, WO96/41802, WO97/03068, WO97/08159, WO97/11695, WO97/11945, WO97/11946, WO97/13512, WO97/16446, WO97/17337, WO97/17338, WO97/17343, WO97/18201, WO97/18202, WO97/18203, WO97/18204, WO97/19073, WO97/38692, WO97/42189, WO97/43281, WO97/45426, WO95/14004, WO98/12183, WO98/14433 and WO98/15545 all incorporated herein by reference. The compounds disclosed in the specifications are useful in the treatment of migraine and cluster headache.

We have now found that administration of a 5HT₁ agonist in conjunction with the COX-2 inhibitor 2-(4-ethoxy-phenyl)-3-(4-methanesulfonyl-phenyl)-pyrazolo[1,5-b]pyridazine compound is beneficial in the treatment of conditions associated with cephalic pain and in alleviating the symptoms associated therewith.

According to one aspect of the invention we therefore provide a method of treating conditions associated with cephalic pain and alleviating the symptoms associated therewith which comprises administering to a mammal, including man, a 5HT₁ agonist or a pharmaceutically acceptable derivative thereof and 2-(4-ethoxy-phenyl)-3-(4-methanesulfonyl-phenyl)-pyrazolo[1,5-b]pyridazine or a pharmaceutically acceptable derivative thereof.

It will be appreciated that reference to treatment is intended to include prophylaxis as well as the alleviation of established symptoms.

According to another aspect of the invention we provide the use of a 5HT₁ agonist or a pharmaceutically acceptable derivative thereof and 2-(4-ethoxy-phenyl)-3-(4-methanesulfonyl-phenyl)-pyrazolo[1,5-b]pyridazine or a pharmaceutically acceptable derivative thereof for the manufacture of a medicament for the treatment of conditions associated with cephalic pain and the alleviation of symptoms associated thereof.

By pharmaceutically acceptable derivative is meant any pharmaceutically acceptable salt, solvate, ester or amide, or salt or solvate of such ester or amide, of the 5HT₁ agonist or the COX-2 inhibitor, or any other compound which upon administration to the recipient is capable of providing (directly or indirectly) the 5HT₁ agonist or the COX-2 inhibitor or an active metabolite or residue thereof.

Of particular interest as pharmaceutical derivatives of the COX-2 inhibitor are compounds modified at the benzenesulphonamide function to provide metabolically labile benzenesulphonamides. Acylated benzenesulphonamide derivatives are of especial interest.

Suitable physiologically acceptable salts according to the invention include acid addition salts formed with inorganic acids such as hydrochlorides, hydrobromides, phosphates and sulphates and with organic acids, for example tatrates, maleates, fumarates, succinates and sulphonates.

Suitable 5HT₁ like receptor agoinsts include sumatriptan, naratriptan, zolmitriptan, rizatriptan, eletriptan, frovatriptan, almotriptan, alniditan, ALX-0646, LY334370, U1092291, IS159 and PNY142633. Naratriptan and sumatriptan are preferred with sumatriptan being particularly preferred. A preferred form of sumatriptan is the succinate salt, particularly the 1:1 succinate.

Therefore in a preferred aspect of the invention there is provided a method of treating conditions associated with cephalic pain and alleviating the symptoms associated therewith which comprises administering to a mammal, including man sumatriptan or a physiologically acceptable salt therefore and a 2-(4-ethoxy-phenyl)-3-(4-methanesulfonyl-phenyl)-pyrazolo[1,5-b]pyridazine or a physiologically acceptable salt thereof.

Compounds for use according to the invention may be administered simultaneously or sequentially and, when administration is sequential, either the 5HT₁ agonist or the COX-2 inhibitor may be administered first.

Compounds for use according to the invention may be administered as the raw material but the active ingredients are preferably provided in the form or pharmaceutical formulations.

The active ingredients may be used either as separate formulations or as a single combined formulation. When combined in the same formulation it will be appreciated that the two compounds must be stable and compatible with each other and the other components of the formulation. Therefore, pharmaceutical formulations comprising a combination as defined above together with a pharmaceutically acceptable diluent or carrier or comprise a further aspect of the invention. When formulated separately they may be provided in any convenient formulation, conveniently in such manner as are known for such compounds in the art.

Accordingly in a further aspect of the invention we provide a pharmaceutical composition which comprises a 5HT₁ agonist or a pharmaceutically acceptable derivative thereof and a COX-2 inhibitor or a pharmaceutically acceptable derivative thereof formulated for administration by any convenient route. Such compositions are preferably in a form adapted for use in medicine, in particular human medicine, and can conveniently be formulated in conventional manner using one or more pharmaceutically acceptable carriers or exciplents.

The formulations include those suitable for oral, parenteral (including subcutaneous e.g. by injection or by depot tablet, intradermal, intrathecal, intramuscular e.g. by depot and intravenous), rectal and topical (including dermal, buccal and sublingual) or in a form suitable for administration by inhalation or insufflation administration although the most suitable route may depend upon for example the condition and disorder of the recipient. The formulations may conveniently be presented in unit dosage form and may be prepared by any of the methods well known in the art of pharmacy. All methods include the step of bringing into association the compounds ("active ingredient") with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation. Preferably such compositions will be formulated for oral administration. It will be appreciated that when the two active ingredients are administered independently, each may be administered by different means.

Formulations suitable for oral administration may be presented as discrete units such as capsules, cachets or tablets (e.g. chewable tablets in particular for paediatric administration) each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a other conventional excipients such as binding agents, (for example, syrup, acacia, gelatin, sorbitol, tragacanth, mucilage of starch, polyvinylpyrrolidone) or hydroxymethyl cellulose or hydroxymethyl cellulose fillers (for example, lactose, sugar, microcrystalline cellulose, maize-starch, calcium phosphate or sorbitol), lubricants (for example, magnesium stearate, stearic acid, talc, polyethylene glycol or silica), disintegrants (for example, potato starch or sodium starch glycollate) or wetting agents, such as sodium lauryl sulfate. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The tablets may be coated according to methods well-known in the art.

Alternatively, the compounds of the present invention may be incorporated into oral liquid preparations such as aqueous or oily suspensions, solutions, emulsions, syrups or elixirs, for example. Moreover, formulations containing these compounds may be presented as a dry product for constitution with water or other suitable vehicle before use. Such liquid preparations may contain conventional additives such as suspending agents such as sorbitol syrup, methyl cellulose, glucose/sugar syrup, gelatin, hydroxyethylcellulose, carboxymethyl cellulose, aluminum stearate gel or hydrogenated edible fats; emulsifying agents such as lecithin, sorbitan mono-oleate or acacia; non-aqueous vehicles (which may include edible oils) such as almond oil, fractionated coconut oil, oily esters, propylene glycol or ethyl alcohol; and preservatives such as methyl or propyl p-hydroxybenzoates or sorbic acid. Such preparations may also be formulated as suppositories, e.g., containing conventional suppository bases such as cocoa butter or other glycerides.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations may be presented in unit-dose or multi-dose containers, for example sealed ampoules and vials, and may be stored in a freeze-dried (lyophilised) condition requiring only the addition of a sterile liquid carrier, for example, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter, hard fat or polyethylene glycol.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose and acacia.

For topical administration to the epidermis, the compounds may be formulated as creams, gels, oiintments or lotions or as a transdermal patch.

The compounds may also be formulated as depot preparations. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For intranasal administration the compounds of the invention may be used, for example as a liquid spray, as a powder or in the form of drops.

For administration by inhalation the compounds according to the invention are conveniently delivered in the form of an aerosol spray presentation from pressurised packs or a nebuliser, with the use of a suitable propellant, e.g. 1,1,1,2-trifluoroethane (HFA 134A) and 1,1,1,2,3,3,3, - heptapropane (HFA 227), carbon dioxide or other suitable gas. In the case of a pressurised aerosol the dosage until may be determined by providing a valve to deliver a metered amount. Capsules and cartridges of e.g. gelatin for use in an inhaler or insufflator may be formulated containing a powder mix of a compound of the invention and a suitable powder base such as lactose or starch.

In addition to the ingredients particularly mentioned above, the formulations may include other agents conventional in the art having regard to the type of formulation in question, for example those suitable for oral administration may include flavouring agents.

It will be appreciated by those skilled in the art that reference herein to treatment extends to prophylaxis as well as the treatment of established diseases or symptoms. Moreover, it will be appreciated that the amount of a compound of the invention required for use in treatment will vary with the nature of the condition being treated and the age and the condition of the patient and will be ultimately at the discretion of the attendant physician or veterinarian. In general, however, doses employed for adult human treatment will typically be in the range of 0.02-5000 mg per day, preferably 1-1500 mg per day. The desired dose may conveniently be presented in a single dose or as divided doses administered at appropriate intervals, for example as two, three, four or more sub-doses per day. The formulations according to the invention may contain between 0.1-99% of the active ingredient, conveniently from 30-95% for tablets and capsules and 3-50% for liquid preparations.

Pharmaceutical compositions according to the invention may be prepared by conventional techniques. When combined in the same formulation for example, the 5HT₁ agonist or a pharmaceutically acceptable derivative thereof and COX-2 inhibitor or a pharmaceutically acceptable derivative thereof may be admixed together, if desired, with suitable excipients. Tablets may be prepared, for example, by direct compression of such a mixture. Capsules may be prepared, for example by filling the blend together with suitable excipients into gelatin capsules, using a suitable filling machine.

Compositions for use according to the invention may, if desired, be presented in a pack or dispenser device which may contain one or more unit dosage forms containing the active ingredients. The pack may, for example, comprise metal or plastic foil, such as a blister pack. Where the compounds are intended for administration as two separate compositions these may be presented, for example, in the form of a twin pack.

It will be appreciated that the dose at which the 5HT₁ agonist and the COX-2 inhibitor is administered will depend on the age and condition of the patient and the frequency and route of administration and will be at the ultimate discretion of the attendant physician. The active ingredients may conveniently be presented in unit dose form.

A proposed dose of 5HT₁ agonist for administration to man (of approximately 70kg body weight) is 0.001 mg to 500 mg per unit dose which may be administered for example 1 to 4 times per day.

The COX-2 inhibitor may conveniently be administered at doses within the normal range taught in the art at which the compounds are therapeutically effective. For example, a proposed dose of the COX-2 inhibitor for use according to the invention is 0.001 to 500mg, preferably 0.01 to 100mg, most preferably 0.05 to 50mg, for example 0.5 to 25mg mg per unit dose, expressed as the weight of free base. The unit dose may be administered in single or divided doses, for example, from 1 to 4 times per day.

## Claims

1. A method of treating conditions associated with cephalic pain and alleviating the symptoms associated therewith which comprises administering to a mammal, including man, sumatriptan or a pharmaceutically acceptable salt or solvate thereof and 2-(4-ethoxy-phenyl)-3-(4-methanesulfonyl-phenyl)-pyrazolo[1,5-b]pyridazine or a pharmaceutically acceptable salt or solvate thereof.

2. A method according to claim 1 wherein the sumatriptan is present as the succinate salt.

3. A pharmaceutical composition which comprises sumatriptan or a pharmaceutically acceptable salt or solvate thereof and 2-(4-ethoxy-phenyl)-3-(4-methanesulfonyl-phenyl)-pyrazolo[1,5-b]pyridazine or a pharmaceutically acceptable salt or solvate thereof.

4. A composition according to claim 3 wherein the sumatriptan is present as the succinate salt.

5. A pharmaceutical composition according to claim 3 or claim 4 adapted for oral administration.
